Europäisches Patentamt

European Patent Office

Office européen des brevets

(11) Publication number: **0 480 594 A2**

# EUROPEAN PATENT APPLICATION

(21) Application number: **91308629.4**

(22) Date of filing: **23.09.91**

(51) Int. Cl.5: **C07C 5/48**, C07C 51/215, B01J 23/36

(30) Priority: **11.10.90 GB 9022127**

(43) Date of publication of application: **15.04.92 Bulletin 92/16**

(84) Designated Contracting States:
**AT BE CH DE DK ES FR GB GR IT LI LU NL SE**

(71) Applicant: **BP Chemicals Limited Belgrave House 76 Buckingham Palace Road London, SW1W 0SU(GB)**

(72) Inventor: **Hallett, Christopher BP Chemicals Limited, Salt End Hedon, Hull HU12 8DS(GB)**

(74) Representative: **Barlow, Michael Thomas et al BP INTERNATIONAL LIMITED Patents & Agreements Division Chertsey Road Sunbury-on-Thames Middlesex TW16 7LN(GB)**

(54) Process and catalyst for the production of ethylene and acetic acid.

(57) A process and catalyst for the production of ethylene and/or acetic acid by oxidation of ethane and/or ethylene with a molecular oxygen-containing gas in the presence of a catalyst composition comprising the elements (A) tungsten, (B) vanadium, (C) rhenium and (D) at least one element selected from the group consisting of lithium, sodium, potassium, rubidium and cesium preferably lithium, the elements being present in combination with oxygen.

EP 0 480 594 A2

This invention relates to a process and catalyst for the production of ethylene and/or acetic acid.

The catalytic oxidative dehydrogenation of ethane to ethylene and/or acetic acid has been described in a number of patent publications, representative of which are US patent 4,250,346 and European patent publication EP 0294845A both of which relate to processes using molybdenum based catalysts.

Our European patent application number 90307052.2 published after the priority date of this present application as EP-A-0407091 relates to a process for producing ethylene and/or acetic acid from ethane and/or ethylene by oxidative dehydrogenation using a catalyst in which molybdenum has been replaced in whole or in part by rhenium or a combination of rhenium and tungsten.

In EP-A-0407091 suitably the catalyst composition comprises the elements A, X and Y in combination with oxygen, the gram-atom ratios of the elements A:X:Y being a:b:c, wherein $A = Mo_dRe_eW_f$; X = Cr, Mn, Nb, Ta, Ti, v and/or W, and preferably Mn, Nb, V and/or W; Y = Bi, Ce, Co, Cu, Fe, K, Mg, Ni, P, Pb, Sb, Si, Sn, Tl and/or U and preferably Sb, Ce, and/or U; a = 1; b = 0 to 2 preferably 0.05 to 1.0; c = 0 to 2, preferably 0.001 to 1.0 and more preferably 0.05 to 1.0 with the proviso that the total value of c for Co, Ni and/or Fe is less than 0.5; d + e + f = a; d is either zero or greater than zero; e is greater than zero and f is either zero or greater than zero. According to EP-A-0407091 preferred catalyst compositions comprise the elements A, Nb, Sb and Z in combination with oxygen, the gram-atom ratios of the elements A:Nb:Sb:Z being a:b:c:g, wherein A is the same as hereinbefore defined; Z is at least one of Ca, Ru and Ga, preferably Ca; a, b and c are the same as hereinbefore defined, and g is 0 to 2 preferably greater than zero.

According to EP-A-0407091 more preferred catalyst compositions comprise the elements A, V, Nb, Sb and Z in combination with oxygen, the gram-atom ratios of the elements A:V:Nb:Sb:Z being a:h:b:c:g wherein A and Z are the same as hereinbefore defined; a, b, c and g are the same as hereinbefore defined, and h is 0 to 1.0.

US patent number US 4,042,533 describes a catalyst for the oxidation of acrolein and methacrolein to acrylic and methacrylic acids respectively in which the catalyst has the empirical formula $Mo_aV_bW_cX_dY_eO_f$ wherein X is one or more of the elements selected from the group consisting of rhenium and titanium and Y is one or more of the elements selected from the group consisting of manganese, iron, copper, tin, zinc, aluminium, cobalt, nickel, phosphorus, cadmium, bismuth, silver, niobium, arsenic, chromium, the alkali and alkaline earth elements. US 4,042,533 does not disclose any examples of catalysts comprising, in combination with oxygen, the elements vanadium, tungsten and rhenium together with alkali elements. The alkali elements are included in US 4,042,533 only as one option in a large list of possible elements, including alkaline earth elements. There is no indication that the alkali elements will be particularly beneficial in catalyst composition comprising rhenium, tungsten and vanadium.

It has now been found that certain alkali metals in particular lithium are beneficial to ethane/ethylene oxidation catalyst compositions comprising tungsten, vanadium and rhenium in combination with oxygen.

Thus, according to the present invention there is provided a process for the production of ethylene and/or acetic acid comprising contacting gaseous ethane and/or ethylene and a molecular oxygen-containing gas at elevated temperature with a catalyst composition comprising the elements (A) tungsten, (B) vanadium, (C) rhenium and (D) at least one element selected from the group consisting of lithium, sodium, potassium, rubidium and cesium, preferably lithium, the elements being present in combination with oxygen.

Also, according to the present invention there is provided a catalyst composition comprising the elements (A) tungsten, (B) vanadium, (C) rhenium and (D) at least one element selected from the group consisting of lithium, sodium, potassium, rubidium and cesium, preferably lithium, the elements being present in combination with oxygen.

Preferably, the relative gram-atom ratios of the elements in the catalyst composition (A):(B):(C):(D) are a:b:c:d where:

a =    0.01 to 1.0, preferably about 0.4 to 0.6, more preferably about 0.5,

b =    0.001 to 1.0, preferably about 0.2 to 0.4, more preferably about 0.3,

c =    0.001 to 1.0, preferably about 0.1 to 0.5,

d =    0.001 to 1.0, preferably about 0.01 to 0.5, and more preferably about 0.02 to 0.14,
        and a + b + c + d = 0.5 to 1.0, preferably about 0.7 to 1.0.

The catalyst composition may comprise additional elements present in combination with oxygen, for example one or more elements selected from the group consisiting of antimony, niobium, molybdenum, tantalum, titanium, bismuth, cobalt, copper, iron, nickel phosphorous, lead, silicon and tin, preferably antimony and/or niobium.

Suitable catalyst compositions have the following nominal empirical formulae based upon the weights of reagents used in their preparation, the element being present in combination with oxygen:

(I) $W_{0.46} Re_{0.111} V_{0.240} Nb_{0.064} Sb_{0.03} K_{0.10}$

2

(II) $W_{0.48}$ $Re_{0.117}$ $V_{0.254}$ $Nb_{0.067}$ $Sb_{0.03}$ $Li_{0.05}$

(III) $W_{0.50}$ $Re_{0.121}$ $V_{0.262}$ $Nb_{0.070}$ $Sb_{0.03}$ $K_{0.02}$

(IV) $W_{0.46}$ $Re_{0.111}$ $V_{0.240}$ $Nb_{0.064}$ $Sb_{0.03}$ $Li_{0.10}$

(V) $W_{0.50}$ $Re_{0.121}$ $V_{0.262}$ $Nb_{0.070}$ $Sb_{0.03}$ $Li_{0.02}$

(VI) $W_{0.48}$ $Re_{0.117}$ $V_{0.254}$ $Nb_{0.067}$ $Sb_{0.03}$ $K_{0.05}$

(VIII) $W_{0.44}$ $Re_{0.11}$ $V_{0.23}$ $Hb_{0.06}$ $Sb_{0.03}$ $Li_{0.14}$

(X) $W_a Re_c V_b$ $Nb_{0.01-0.2}$ $Sb_{0.005-0.2}$ $D_d$

wherein a,b,c,d and D are as hereinbefore defined.

The catalyst composition may be prepared by any of the methods conventionally employed for the preparation of catalysts. Suitably the catalyst may be prepared from a solution of soluble compounds and/or complexes and/or compounds of each of the metals. The solution is preferably an aqueous solution having a pH in the range from 1 to 14, preferably from 2 to 8, at a temperature of from 20° to 100°C.

Generally, a mixture of compounds containing the elements is prepared by dissolving sufficient quantities of soluble compounds and dispersing any insoluble compounds so as to provide a desired gram-atom ratio of the elements in the catalyst composition. The catalyst composition may then be prepared by removing the solvent from the mixture. The catalyst may be calcined by heating to a temperature of from 200 to 550°C, suitably in air or oxygen, for a period of from 1 minute to 24 hours. Preferably, the air or oxygen is slowly flowing.

Suitable compounds for use in the preparation of the catalyst compositon are: ammonium vanadate as a source of vanadium; tungstic acid or ammonium tungstate as a source of tungsten; niobium oxalate as a source of niobium; antimony acetate as a source of antimony; lithium or potassium nitrate as a source of lithium and potassium respectively and ammonium rhenate as a source of rhenium.

The catalyst may be used unsupported or supported. Suitable supports include silica, alumina, zirconia, titania, silicon carbide and mixtures of two or more thereof.

The catalyst may be used in the form of a fixed or a fluidised bed.

The feed gas to the process of the present invention comprises ethane and/or ethylene, preferably ethane. Ethane produces ethylene and optionally acetic acid. Ethylene produces acetic acid.

In a process for producing acetic acid by oxidation of ethane, unreacted ethane and ethylene product may be separated from acetic acid product and recycled as co-feed with additional ethane and oxygen to the catalyst.

Ethane and/or ethylene may be used in substantially pure form or admixed with one or more of nitrogen, methane, carbon dioxide and water in the form of steam, which may be present in major amounts, for example greater than 5 volume percent or one or more of hydrogen, carbon monoxide, $C_3/C_4$ alkenes and alkenes, which may be present in minor amounts, for example less than 5 volume percent.

The molecular oxygen-containing gas may be air or a gas richer or poorer in molecular oxygen than air, for example oxygen. A suitable gas may be, for example, oxygen diluted with a suitable diluent, for example nitrogen or helium.

In the process of the present invention water (steam) may be fed in addition to ethane and/or ethylene and the molecular oxygen-containing gas, because this can improve the selectivity to acetic acid if required.

The elevated temperature may suitably be in the range from 100 to 600°C, preferably from 200 to 500°C.

The pressure may suitably be atmospheric or superatmospheric, for example in the range from 1 to 50 bar, preferably from 1 to 40 bar.

For fixed bed reactors, if the pressure is at least 14 barg the temperature is preferably less than 400°C.

Operating conditions and other information applicable to the performance of the process of the present invention may be found in the aforesaid prior art, for example US Patent No. 4,250,346.

The process of the present invention will now be further illustrated by reference to the following Examples.

CATALYST PREPARATION

In the examples the catalyst compositions are expressed as nominal empirical formulae in terms of relative gram-atoms of elements based upon the weights of reagents used. It will be understood that the elements are present in combination with oxygen.

PREPARATION OF CATALYSTS ACCORDING TO THE PRESENT INVENTION

Example 1 Preparation of Catalyst I (Ref. 630/37)

A catalyst having the nominal empirical formula:

(I) $W_{0.46} Re_{0.111} V_{0.240} Nb_{0.064} Sb_{0.03} K_{0.10}$

was prepared as follows.

A first mixture, A was prepared by partially dissolving and dispersing ammonium vanadate (4.19g, Alrich) in deionised water (50ml) in a beaker. A second mixture, B was prepared by partially dissolving niobium oxalate (5.18g, Atomergic Chemetals) in deionised water (25 ml) in a beaker. To this was added antimony acetate (1.23g, Ventron) and potassium nitrate (1.52 g, Aldrich) together with more deionised water (25 ml). A third mixture, C was prepared by partially dissolving ammonium rhenate (4.43g, Johnson Matthey) in deionised water (25 ml) and dispersing therein tungstic acid (17.2 g, Aldrich) and further deionised water (25 ml). Mixtures A and B were heated separately with stirring for 15 minutes to between 50 and 80°C. Both beakers contained undissolved solid and/or suspension after this time. Mixture A was added to mixture B and the whole heated with stirring for 15 minutes. During this time mixture C was heated with stirring to 50-80°C. After mixture C and the combination of mixtures A and B had been heated for 15 minutes, mixture C was added to the combination of mixtures A and B which was at about 70°C. The whole was heated and stirred for a further 15 minutes at about 70°C before being brought to boiling point and the water evaporated off to leave a slurry. The slurry was dried at 115°C for 16 hours in an oven. The material was then sieved to 2.0mm to 0. 18mm before being heated at 2 degrees/min to 350°C under a flow of air (5-10ml/minute) and calcined at 410°C for 4 hours. Different calcination temperatures were used when this method was used for the preparation of other catalysts. The air flow was then stopped and the material cooled at 2 degrees per minute to 80°C to 100°C and then by natural cooling. The product catalyst composition was sieved to 500 - 180 microns for use in the catalyst testing procedures.

Example 2 Preparation of Catalyst II (Ref. 630/38)

A catalyst having the nominal empirical formula:

(II) $W_{0.48} Re_{0.117} V_{0.254} Nb_{0.067} Sb_{0.03} Li_{0.05}$ was prepared according to the same method as Example 1 using lithium nitrate (Aldrich) in place of potassium nitrate and with the following quantities of reagents:

| | |
|---|---|
| ammonium vanadate | 4.41 g |
| niobium oxalate | 5.50 g |
| antimony acetate | 1.33 g |
| lithium nitrate | 0.51 g |
| ammonium rhenate | 4.71 g |
| tungstic acid | 18.18 g |

A calcination temperature of 380°C was used.

Example 3 Preparation of Catalyst III (Ref 630/40)

A catalyst having the nominal empirical formula:

(III) $W_{0.50} Re_{0.121} V_{0.262} Nb_{0.070} Sb_{0.03} K_{0.02}$

was prepared according to the same method as Example 1 by dispersing ammonium tungstate (Johnson Matthey) in place of tungstic acid and with the following quantities of reagents:

| | |
|---|---|
| ammonium vanadate | 4.57 g |
| niobium oxalate | 5.65 g |
| antimony acetate | 1.34 g |
| potassium nitrate | 0.31 g |
| ammonium rhenate | 4.84 g |
| ammonium tungstate | 19.59 g |

A calcination temperature of 380°C was used.

Example 4 Preparation of Catalyst IV (Ref 630/41)

A catalyst having the nominal empirical formula:

4

(IV) $W_{0.46}$ $Re_{0.111}$ $V_{0.240}$ $Nb_{0.064}$ $Sb_{0.03}$ $Li_{0.10}$

was prepared according to the same method as Example 3 using lithium nitrate in place of potassium nitrate and with the following quantities of reagents:

| ammonium vanadate | 4.19 g |
|---|---|
| niobium oxalate | 5.18 g |
| antimony acetate | 1.23 g |
| lithium nitrate | 1.04 g |
| ammonium rhenate | 4.43 g |
| ammonium tungstate | 17.98 g |

A calcination temperature of 350°C was used.

Example 5 Preparation of Catalyst V (Ref 630/47)

A catalyst having the nominal empirical formula:
(V) $W_{0.50}$ $Re_{0.121}$ $V_{0.262}$ $Nb_{0.070}$ $Sb_{0.03}$ $Li_{0.02}$
was prepared as follows:

Mixtures A, B and C were prepared as in Example 1 using lithium nitrate in place of potassium nitrate and ammonium tungstrate in place of tungstic acid and with the following quantities of reagents.

| ammonium vanadate | 4.57 g |
|---|---|
| niobium oxalate | 5.64 g |
| antimony acetate | 1.34 g |
| lithium nitrate | 0.20 g |
| ammonium rhenate | 4.83 g |
| ammonium tungstate | 19.57 g |

Mixtures A and B were heated to about 87 and 82°C respectively with stirring for 15 minutes. Mixture A was added to mixture B and stirring of mixture C was commenced. Within 1-2 minutes addition of hydrogen peroxide (70 ml, 30 vol) was started to mixture C and this was completed over 1 minute. This caused the solid in mixture C to dissolve giving an almost colourless solution. After heating mixture C to 95°C and the combination of mixtures A and B for 15 minutes, mixture C was added to mixture A/B which was at about 80°C in aliquots over 2 minutes (to avoid a vigorous reaction). The combined mixture was then held at about 80°C for 10 minutes before the water was evaporated off to leave a slurry which was dried at 115°C for 16 hours. The material was sieved to 2.0mm to 0.18mm, heated to 350°C at 2 degree per minute under flowing air (about 5.5 ml/minute) and calcined at 410°C for 4 hours. Different calcination temperature were used when this method was used for the preparation of other catalysts. After this time the air flow was stopped and the material cooled to below 100°C at a rate of 2 degree per min before being allowed to cool naturally. The catalyst composition was then sieved to 500 to 180 microns for use in the catalyst test procedures.

Example 6 Preparation of Catalyst VI (Ref 630/50)

A catalyst having the nominal empirical formula:
(VI) $W_{0.48}$ $Re_{0.117}$ $V_{0.254}$ $Nb_{0.067}$ $Sb_{0.03}$ $K_{0.05}$
was prepared according to the same method as Example 5 using potassium nitrate in place of lithium nitrate and with the following quantities of reagents:

| ammonium vanadate | 4.43 g |
|---|---|
| niobium oxalate | 5.48 g |
| antimony acetate | 1.31 g |
| potassium nitrate | 0.76 g |
| ammonium rhenate | 4.68 g |
| ammonium tungstate | 18.97 g |

A calcination temperature of 350°C was used.

Example 7 Preparation of Catalyst VII (Ref 630/51)

Example 6 was repeated to give a catalyst having a nominal empirical formula (VI) $W_{0.48}$ $Re_{0.117}$ $V_{0.254}$ $Nb_{0.067}$ $Sb_{0.03}$ $K_{0.05}$.

Example 8 Preparation of Catalyst VIII (Ref 630/53)

A catalyst having the nominal empirical formula:
(VIII) $W_{0.44}$ $Re_{0.11}$ $V_{0.23}$ $Nb_{0.06}$ $Sb_{0.03}$ $Li_{0.14}$
was prepared according to the same method as Example 4 but with the following quantities of reagents:

| ammonium vanadate | 3.99g |
|---|---|
| niobium oxalate | 4.96g |
| antimony acetate | 1.18g |
| ammonium rhenate | 4.24g |
| ammonium tungstate | 17.17g |
| lithium nitrate | 1.47g |

A calcination temperature of 350°C was used.

PREPARATION OF COMPARATIVE EXAMPLE CATALYSTS

Comparative Example A - Preparation of Catalyst A (Ref 630/42)

A catalyst having the nominal empirical formula:
(A) $W_{0.49}$ $Re_{0.12}$ $V_{0.25}$ $Nb_{0.07}$ $Sb_{0.03}$ $Ca_{0.05}$
was prepared according to the same method as Example 3 using calcium nitrate in place of potassium nitrate and with the following quantities of reagent:

| ammonium vanadate | 4.43 g |
|---|---|
| niobium oxalate | 5.47 g |
| antimony acetate | 1.30 g |
| calcium nitrate | 1.78 g |
| ammonium rhenate | 4.68 g |
| ammonium tungstate | 19.00 g |

A calcination temperature of 410°C was used.

Comparative Example B - Preparation of Catalyst B (Ref 630/39)

A catalyst having the nominal empirical formula:
(B) $W_{0.50}$ $Re_{0.121}$ $V_{0.262}$ $Nb_{0.070}$ $Sb_{0.03}$ $Ca_{0.02}$
was prepared according to the same method as Example 1 but using calcium nitrate in place of potassium nitrate and with the following quantities of reagents:

6

| ammonium vanadate | 4.56 g |
| --- | --- |
| niobium oxalate | 5.65 g |
| antimony acetate | 1.35 g |
| calcium nitrate | 0.72 g |
| ammonium rhenate | 4.83 g |
| tungstic acid | 18.73 g |

A calcination temperature of 350°C was used.

Comparative Example C - Preparation of Catalyst C (Ref 630/46)

A catalyst having the nominal empirical formula:
(C) $W_{0.570}$ $V_{0.24}$ $Nb_{0.064}$ $Sb_{0.03}$ $Li_{0.10}$
was prepared according to the same method as Example 4 and with the following quantities of reagents:

| ammonium vanadate | 4.19 g |
| --- | --- |
| niobium oxalate | 5.18 g |
| antimony acetate | 1.24 g |
| lithium nitrate | 1.04 g |
| ammonium tungstate | 22.21 g |

A calcination temperature of 350°C was used.

Comparative Example D - Preparation of Catalyst D (Ref 630/48 and 630/48R)

A catalyst having the nominal empirical formula:
(D) $W_{0.46}$ $Re_{0.111}$ $V_{0.240}$ $Nb_{0/064}$ $Sb_{0.03}$ $Ca_{0.10}$
was prepared according to the same method as Example 5 but using calcium nitrate in place of lithium nitrate and with the following quantities of reagents:

| ammonium vanadate | 4.19 g |
| --- | --- |
| niobium oxalate | 5.19 g |
| antimony acetate | 1.24 g |
| calcium nitrate | 3.54 g |
| ammonium rhenate | 4.43 g |
| ammonium tungstate | 17.98 g |

A calcination temperature of 380°C was used.

Comparative Example E Preparation of Catalyst E (Ref 630/49)

A catalyst having the nominal empirical formula:
(E) $W_{0.51}$ $Re_{0.12}$ $V_{0.26}$ $Nb_{0.07}$ $Sb_{0.03}$
was prepared according to the same method as Example 3 using no potassium nitrate and with the following quantities of reagents:

| ammonium vanadate | 4.66g |
| --- | --- |
| niobium oxalate | 5.76g |
| antimony acetate | 1.37g |
| ammonium rhenate | 4.92g |
| ammonium tungstate | 19.97g |

A calcination temperature of 350°C was used.

Comparative Example F Preparation of Catalyst F (Ref 630/63)

A catalyst having the following nominal empirical formula:

(F) $W_{0.46}$ $Re_{0.11}$ $V_{0.24}$ $Nb_{0.06}$ $Sb_{0.03}$ $Mg_{0.1}$

was prepared according to the same method as Example 3 but using magnesium acetate in place of potassium nitrate and with the following quantities of reagents:

| | |
|---|---|
| ammonium vanadate | 4.18g |
| niobium oxalate | 5.18g |
| antimony acetate | 1.23g |
| ammonium rhenate | 4.43g |
| ammonium tungstate | 17.99g |
| magnesium acetate | 3.21g |

## CATALYST TESTING

In the Examples the following terms are used:-

$$GHSV = Gas\ Hourly\ Space\ Velocity$$

$$= \frac{\underline{Volume\ of\ gas\ flowing\ through\ catalyst\ bed\ (ml/hr\ at\ NTP)}}{Volume\ of\ catalyst\ bed\ (ml).}$$

$$Ethane\ conversion\ (\%) = 100\ \times\ \frac{[CO]/_2+[CO_2]/_2+[C_2H_4]+[CH_3COOH]}{[CO]/_2+[CO_2]/_2+[C_2H_4]+[C_2H_6]+[CH_3COOH]}$$

$$Selectivity\ to\ ethylene\ (\%) = 100\ \times\ \frac{[C_2H_4]}{[CO]/_2+[CO_2]/_2+[C_2H_4]+[CH_3COOH]}$$

$$Selectivity\ to\ acetic\ acid\ (\%) = 100\ \times\ \frac{[CH_3COOH]}{[CO]/_2+[CO_2]/_2+[C_2H_4]+[CH_3COOH]}$$

$$Selectivity\ to\ carbon\ monoxide\ (\%) = 100\ \times\ \frac{[CO]/_2}{[CO]/_2 + [CO_2]/_2 + [C_2H_4] + [CH_3COOH]}$$

$$Selectivity\ to\ carbon\ dioxide\ (\%) = 100\ \times\ \frac{[CO_2]/_2}{[CO]/_2 + [CO_2]/_2 + [C_2H_4] + [CH_3COOH]}$$

wherein [ ] = concentrations in mol%

and $[C_2H_6]$ = concentration of unconverted ethane.

## CATALYST TESTING METHOD

1ml of catalyst was loaded into a corrosion resistant stainless steel tube and the reactor tube assembly placed in a furnace. The ethane:oxygen:helium feed gas ratio was then set up in a gas mixing manifold and allowed to pass over the catalyst and the pressure was adjusted to the required value (14 barg) using a back pressure regulator. The product vapours and gases leaving the reactor were sampled and analysed by gas-liquid chromatography (GLC). The catalyst was then heated to 260°C. The temperature was measured by means of a thermocouple on the outside surface of the reactor. Then the reactor temperature was increased in stages, with data being collected by GLC at different temperatures, until the oxygen concentration in the product gas was about 0.3% to 0.6% vol/vol. Then steady conditions were established for 8 hours at this final temperature during which further analyses of the product gas were undertaken by GLC.

GLC SPECIFICATION:

Gas Analysis: 3m Carbosieve S2 Column & Molecular Sieve Column.
Liquid Analysis: 2.5m CarboPack B/Peg 20M Column

REACTION CONDITIONS

Reactor Pressure: 14 barg
GHSV : approx. 3000 hr$^{-1}$
Feed Composition (by volume) : 71% Ethane, 6.5% Oxygen, 23% Helium
Reactor Temperature: In the range 250-385°C
Catalyst Particle Size: 0.18 - 0.5 mm diameter.

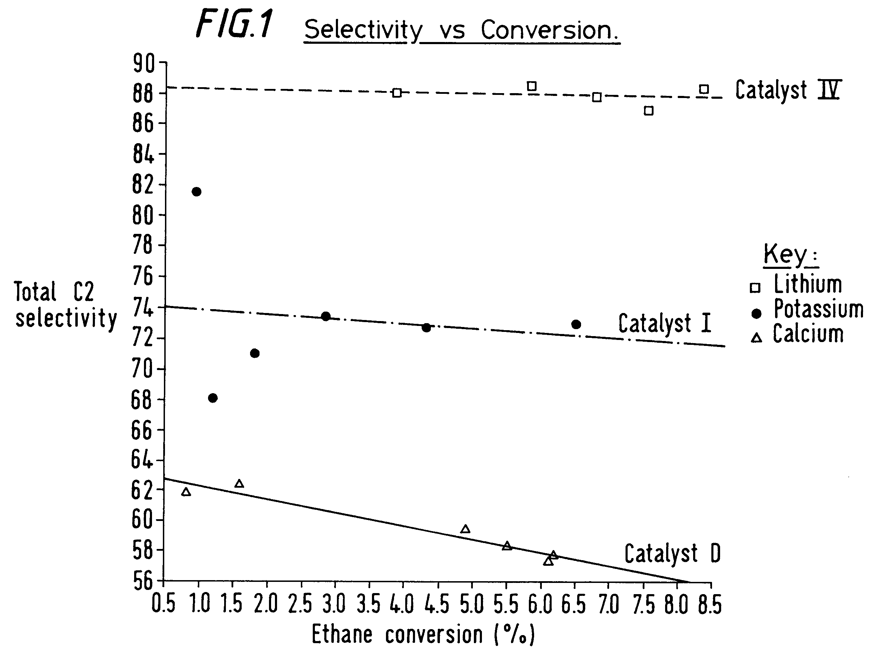

The results based upon average analyses at the final reaction temperature are shown in Tables 1 and 2. Reactions using catalyst D was repeated as DR and using catalyst VI was repeated twice as VIR and VIR2. Results at intermediate temperatures (and hence intermediate ethane conversion) are shown in graph form in Figure 1 for catalyst I, IV and D.

Referring to Figure 1 which shows the operating lines of selectivities to $C_2$ products (ethylene plus acetic acid) versus ethane conversion, it will be seen that catalysts I and IV have higher selectivity than catalyst D at the same conversion of ethane. Also catalyst IV (comprising lithium) has a higher selectivity than catalyst I (comprising potassium) at the same ethane conversion.

Referring to Tables 1 and 2, the selectivities to ethylene and acetic acid are greater for catalyst IV (with rhenium) than those of catalyst C (no rhenium).

Referring to Tables 1 and 2, although the results for comparative Example E show a higher acetic acid selectively than for Examples VI, VIR, VIR2 and VII, containing potassium, the total selectivity to useful products (ethylene plus acetic acid) is less than the average for these catalysts with potassium. Similarly the selectivities to ethylene plus acetic acid for Examples I and V may be adversely affected by the higher calcination temperatures used than for other catalysts.

The Tables also show alkaline earth elements such as magnesium and calcium to be detrimental to catalyst performance.

The Tables also show that catalyst IV (10 g.eq. % lithium) has a higher selectivity to ethylene and acetic acid than catalyst VIII (14 g.eq. % lithium) implying for lithium at least, a maximum to the promoter's effect in the range of 2 to 14 gram equivalent % of the elements present in combination with oxygen.

A further test using 3ml of catalyst IV was performed at 24.5 barg with GHSV = 4000 and with water co-feed through a preheater at 0.27g per litre of gas in the stream. At 330°C the following data were obtained.

| | |
|---|---|
| Ethane conversion | 8.2% |
| Ethylene selectivity | 46.9% |
| Acetic and selectivity | 37.2% |
| CO selectivity | 4.6% |
| $CO_2$ selectivity | 3.75% |
| Acetaldehyde selectivity | 1.4% |
| Ethanol selectivity | 3% |
| Ethyl acetate selectivity | 3% |

This shows the increase in selectivity to acetic acid due to the presence of water cofeed and increased pressure.

TABLE 1

Comparative Examples

| Example No. | Catalyst Ref No. | Calcination Temperature (°C) | Reaction Temperature (°C) | Ethane Conversion (%) | Selectivity To Ethylene (%) | Selectivity To Acetic Acid (%) | Selectivity To CO (%) | Selectivity To $CO_2$ (%) |
|---|---|---|---|---|---|---|---|---|
| I | 630/37 | 410 | 385 | 6.49 | 70.7 | 2.29 | 11.7 | 15.3 |
| II | 630/38 | 380 | 326 | 6.84 | 66.3 | 10.93 | 9.99 | 12.8 |
| III | 630/40 | 380 | 362 | 6.90 | 70.5 | 4.81 | 10.4 | 14.2 |
| IV | 630/41 | 350 | 310 | 8.34 | 79.9 | 8.65 | 5.04 | 6.39 |
| V | 630/47 | 410 | 335 | 7.71 | 67.1 | 4.83 | 12.2 | 15.9 |
| VI | 630/50 | 350 | 321 | 7.43 | 60.6 | 14.8 | 12.7 | 11.9 |
| VIR | 630/50 | 350 | 325 | 6.60 | 62.6 | 13.2 | 12.7 | 11.5 |
| VIR2 | 630/50 | 350 | 322 | 6.40 | 65.6 | 12.8 | 11.9 | 9.7 |
| VII | 630/51 | 350 | 320 | 7.90 | 67.3 | 14.9 | 9.0 | 8.8 |
| VIII | 630/53 | 350 | 333 | 7.80 | 72.3 | 7.5 | 8.6 | 11.6 |

EP 0 480 594 A2

TABLE 2

Comparative Examples

| Comparative Example No. | Catalyst Ref No. | Calcination Temperature (°C) | Reaction Temperature (°C) | Ethane Conversion (%) | Selectivity To Ethylene (%) | Selectivity To Acetic Acid (%) | Selectivity To CO (%) | Selectivity To $CO_2$ (%) |
|---|---|---|---|---|---|---|---|---|
| A | 630/42 | 410 | 356 | 5.47 | 53.6 | 2.94 | 23.9 | 19.5 |
| B | 630/39 | 350 | 332 | 5.16 | 48.6 | 5.89 | 20.7 | 23.1 |
| C | 630/46 | 350 | 346 | 5.82 | 46.6 | 2.05 | 28.2 | 20.0 |
| D | 630/48 | 380 | 352 | 6.17 | 54.1 | 3.67 | 22.8 | 19.4 |
| DR | 630/48 | 380 | 351 | 6.10 | 54.0 | 3.40 | 22.6 | 20.0 |
| E | 630/49 | 350 | 325 | 7.18 | 54.8 | 18.9 | 14.9 | 11.4 |
| F | 630/63 | 350 | 324 | 5.70 | 52.5 | 7.9 | 22.2 | 17.5 |

**Claims**

1. A process for the production from gaseous ethane and/or ethylene of a product comprising ethylene and/or acetic acid, by contacting the ethane and/or ethylene and a molecular oxygen-containing gas at elevated temperature with a catalyst composition characterised in that the composition comprises the elements (A) tungsten, (B) vanadium, (C) rhenium (D) at least one element selected from the group consisting of lithium, sodium, potassium, rubidium and cesium, the elements being present in combination with oxygen.

2. A process as claimed in claim 1 characterised in that the element (D) in combination with oxygen comprises lithium.

3. A process as claimed in claim 1 or claim 2 characterised in that the relative gram-atom ratios of the elements (A):(B):(C):(D), present in the catalyst composition in combination with oxygen, are a:b:c:d wherein:-

a = 0.01 to 1.0
b = 0.001 to 1.0
c = 0.001 to 1.0
d = 0.001 to 1.0
and a + b + c + d = 0.5 to 1.0

4. A process as claimed in any one of the preceding claims characterised in that the catalyst composition additionally comprises one or more elements selected from the group consisting of molybdenum, antimony, niobium, tantalum, titanium, bismuth, cobalt, copper, iron, nickel, phosphorous, lead, silicon and tin, the elements being present in combination with oxygen.

5. A process as claimed in claim 4 characterised in that the catalyst composition additionally comprises the elements antimony and/or niobium present in combination with oxygen.

6. A process as claimed in claim 5 characterised in that the catalyst composition has a nominal empirical formula selected from the group consisting of:-

(I) $W_{0.46} Re_{0.111} V_{0.240} Nb_{0.064} Sb_{0.03} K_{0.10}$,

(II) $W_{0.48} Re_{0.117} V_{0.254} Nb_{0.067} Sb_{0.03} Li_{0.05}$,

(III) $W_{0.50} Re_{0.121} V_{0.262} Nb_{0.070} Sb_{0.03} K_{0.02}$,

(IV) $W_{0.46} Re_{0.111} V_{0.240} Nb_{0.064} Sb_{0.03} Li_{0.10}$,

(V) $W_{0.50} Re_{0.121} V_{0.262} Nb_{0.070} Sb_{0.03} Li_{0.02}$,

(VI) $W_{0.48} Re_{0.117} V_{0.254} Nb_{0.067} Sb_{0.03} K_{0.05}$,

(VIII) $W_{0.44} Re_{0.11} V_{0.23} Nb_{0.06} Sb_{0.03} Li_{0.14}$ and

(X) $W_a Re_c V_b Nb_{0.01-0.2} Sb_{0.005-0.2} D_d$,

wherein a, b, c, d and D are as defined in claim 3, the elements being present in combination with oxygen.

7. A process as claimed in any one of the preceding claims characterised in that steam is contacted with the catalyst composition with ethane and/or ethylene and molecular oxygen-containing gas.

8. A catalyst composition characterised in that it comprises the elements (A) tungsten, (B) vanadium, (C) rhenium and (D) at least one element selected from the group consisting of lithium, sodium, potassium, rubidium and cesium, the elements being present in combination with oxygen.

9. A catalyst composition as claimed in claim 8 characterised in that the relative gram-atom ratios of the elements (A):(B):(C):(D) present in the catalyst composition in combination with oxygen, are a:b:c:d wherein:-

a = 0.01 to 1.0,
b = 0.001 to 1.0,
c = 0.01 to 1.0,
d = 0.001 to 1.0,
and a + b + c + d = 0.5 to 1.0.

10. A catalyst composition as claimed in claim 9 characterised in that the element (D) in combination with oxygen comprises lithium.

11. A catalyst composition as claimed in claim 10 characterised in that d is about 0.01 to 0.5.

12

**12.** A catalyst composition as claimed in claim 11 characterised in that d is about 0.02 to 0.14.

**13.** A catalyst composition as claimed in any one of claims 8 to 12 characterised in that the composition additionally comprises one or more elements selected from the group consisting of molybdenum, antimony, niobium, tantalum, titanium, bismuth, cobalt, copper, iron, nickel, phosphorous, lead, silicon and tin, the elements being present in combination with oxygen.

**14.** A catalyst composition as claimed in claim 13 characterised in that the composition additionally comprises the elements antimony and/or niobium present in combination with oxygen.

**15.** A catalyst composition characterised in that it has a nominal empirical formula selected from the group consisting of:-

(I) $W_{0.46}$ $Re_{0.111}$ $V_{0.240}$ $Nb_{0.064}$ $Sb_{0.03}$ $K_{0.10}$,

(II) $W_{0.48}$ $Re_{0.117}$ $V_{0.254}$ $Nb_{0.067}$ $Sb_{0.03}$ $Li_{0.05}$,

(III) $W_{0.50}$ $Re_{0.121}$ $V_{0.262}$ $Nb_{0.070}$ $Sb_{0.03}$ $K_{0.02}$,

(IV) $W_{0.46}$ $Re_{0.111}$ $V_{0.240}$ $Nb_{0.064}$ $Sb_{0.03}$ $Li_{0.10}$,

(V) $W_{0.50}$ $Re_{0.121}$ $V_{0.262}$ $Nb_{0.070}$ $Sb_{0.03}$ $Li_{0.02}$,

(VI) $W_{0.48}$ $Re_{0.117}$ $V_{0.254}$ $Nb_{0.067}$ $Sb_{0.03}$ $K_{0.05}$,

(VIII) $W_{0.44}$ $Re_{0.11}$ $V_{0.23}$ $Nb_{0.06}$ $Sb_{0.03}$ $Li_{0.14}$, and

(X) $W_a Re_c V_b$ $Nb_{0.01-0.2}$ $Sb_{0.005-0.2}$ $D_d$,

wherein a,b,c,d and D are as defined in claim 9, the elements being present in combination with oxygen.

**16.** A catalyst composition comprising a composition as claimed in any one of claims 8 to 15 supported on a support selected from the group consisting of silica, alumina, zirconia, titania, silicon, carbide and mixtures of two or more thereof.

FIG.1 Selectivity vs Conversion.

Catalyst IV

Catalyst I

Catalyst D

Key:
□ Lithium
● Potassium
△ Calcium

Total C2 selectivity

Ethane conversion (%)